# EUROPÄISCHE PATENTSCHRIFT

(11) **EP 0 839 201 B1**
(45) Veröffentlichungstag und Bekanntmachung des Hinweises auf die Patenterteilung: **10.08.2005**
(21) Anmeldenummer: 97923862.3
(22) Anmeldetag: 09.05.1997
(51) Int. Cl.: C12N 15/57, C12N 9/50, C12N 15/11, C12N 15/15, A61K 38/48, C12Q 1/37, A61K 38/55, C12N 15/79

(54) **CATHEPSIN-L, DESSEN PRÄPROFORM UND DAS ENTSPRECHENDE PROPEPTID AUS CILIATEN**
CATHEPSIN-L, THE PRE-PROFORM THEREOF AND THE CORRESPONDING PROPEPTIDE FROM CILIATES
CATHEPSINE-L, SA PRE-PROFORME ET LE PROPEPTIDE CORRESPONDANT OBTENUS A PARTIR DE CILIATES

(30) Priorität: 14.05.1996 DE 19619366
(43) Veröffentlichungstag der Anmeldung: 06.05.1998
(73) Patentinhaber: Nutrinova Nutrition Specialties & Food Ingredients GmbH, 65926 Frankfurt am Main (DE)
(72) Erfinder: KIY, Thomas, D-65929 Frankfurt am Main (DE); SCHULTZ, Joachim, D-72119 Ammerbuch (DE)
(74) Vertreter: Luderschmidt, Schüler & Partner
(86) Internationale Anmeldenummer: PCT/EP1997/002388
(87) Internationale Veröffentlichungsnummer: WO 1997/043425

(56) Entgegenhaltungen:
- VOELKEL H ET AL: "Cathepsin L is an intracellular and extracellular protease in Paramecium tetraurelia: Purification, cloning, sequencing and specific inhibition by its expressed propeptide." EUROPEAN JOURNAL OF BIOCHEMISTRY 238 (1). 1996. 198-206. ISSN: 0014-2956, XP002042623
- A. KOK AND R. PAESTE: "Lysosomal enzymes of Paramecium caudatum and Paramecium tetraurelia" EXPERIMENTAL CELL RESEARCH, Bd. 139, Nr. 1, Mai 1982, Seiten 159-169, XP002043164
- E. KESSLER AND M. SAFRIN: "The propeptide of Pseudomonas aeruginosa elastase acts as an elastase inibitor" JOURNAL OF BIOLOGICAL CHEMISTRY, Bd. 269, Nr. 36, 9.September 1994, MD US, Seiten 22726-22731, XP002042624 in der Anmeldung erwähnt

## Beschreibung

Die Erfindung betrifft die Gewinnung der Präproform des Cathepsin-L, deren Leadersequenz, des Cathepsin-L und des dazugehörigen Propeptid aus Ciliaten - insbesondere Paramecium, die Verwendung dieser Peptide und ein Verfahren zur Herstellung des Cathepsin-L aus Ciliaten.

Der Befund, daß Propeptide verschiedener Proteasen - nachdem sie bei der Aktivierung der Protease-Zymogene freigesetzt wurden - als Proteaseinhibitoren wirken können, ist bekannt. Das Propeptid der Elastase aus Pseudomonas aeruginosa etwa lagert sich nach der Abspaltung an die Elastase und führt damit zur Inaktivierung des Enzyms (Kessler & Safrin, 1994, J. Biol. Chem., 269, 22726). Die Propeptide des Papains und der Papaya-Proteinase IV wirken selektiv als Inhibitoren der reifen Papaya-Proteasen und der verwandten Cathepsine B und L aus der Rattenleber (Taylor et al., 1995, Biochem. Soc. Trans., 23, 80). Auch die Propeptide weiterer Cathepsine können als Proteaseninhibitoren wirken. So inhibiert das synthetisch hergestellte Propeptid des humanen Procathepsin-D bovines Cathepsin D (Vagner et al., 1993, Collect. Czech. Chem. Commun., 58, 435).

Cathepsin-L, eine Protease, spielt eine wichtige Rolle bei verschiedenen Krankheitsbildern. So ist dieses Enzym wahrscheinlich von entscheidender Bedeutung bei der Invasivität von Tumoren und der Bildung von Metastasen (Pike, 1991, Dissertation Abstr. Intern., 53, 4645). Auch beim Eindringen pathogener Bakterien oder parasitischer Protozoen in das Wirtsgewebe kann diese Protease beteiligt sein. Weiterhin ist Cathepsin-L an der Degradation der Knochenmatrix beteiligt. Bei der Behandlung von Osteoporose erscheint dieses Enzym daher als lohnendes Target (Pharma Japan, Sept. 1995, 1468, 23).

Schließlich sei erwähnt, daß Cathepsin-L auch an der Ausprägung inflammatorischer Krankheiten wie Arthritis beteiligt ist.

Die Identifizierung geeigneter Cathepsin-L-Inhibitoren könnte ein wichtiger Schritt bei der Entwicklung geeigneter Präparate für die Therapie der genannten Erkrankungen darstellen. Weiterhin wäre auch eine geeignete Quelle zur Gewinnung größerer Mengen von Cathepsin-L von großem Vorteil. Das Enzym ließe sich in Screening-Systemen zur Auffindung geeigneter Protease-Inhibitoren einsetzen. Darüber hinaus ließe es sich z.B. in Wundsalben einsetzen, wo es den Abbau nekrotischen Gewebes katalysieren könnte.

Die vorliegende Erfindung betrifft demgemäß eine Cathepsin-L-Präproform, erhältlich aus Ciliaten, vorzugsweise aus Paramecium, besonders bevorzugt aus Paramecium tetraurelia, und die für ein solches Protein kodierende DNA-Sequenz.

Die Erfindung betrifft ferner ein Cathepsin-L aus Ciliaten, vorzugsweise aus Paramecium, besonders bevorzugt aus Paramecium tetraurelia und die zugehörige DNA-Sequenz, ein Verfahren zu dessen Herstellung aus Ciliaten, sowie dessen Verwendung zur Herstellung eines Arzneimittels zur Behandlung von Wunden.

Das Cathepsin-L gemäß der vorliegenden Erfindung kann ferner Anwendung zur Identifizierung geeigneter Inhibitoren - beispielsweise durch sog. Molecular Modeling dienen.

Ferner wird durch die vorliegende Erfindung ein Cathepsin-L-Propeptid und dessen DNA-Sequenz aus Ciliaten, vorzugsweise aus Paramecium, besonders bevorzugt aus Paramecium tetraurelia bereitgestellt.
Das Propeptid des Cathepsin-L aus Ciliaten ist ein hochspezifischer Inhibitor desselben und eignet sich demgemäß zur Herstellung von Arzneimitteln zur Behandlung von inflammatorischen Erkrankungen, metastasierenden Tumoren, bakteriellen Infektionen, Infektionen mit parasitären Protozoen oder der Osteporose.

Die vorliegende Erfindung stellt ferner eine Präsequenz, entsprechend der Leader- oder Signalsequenz des Cathepsin-L aus Ciliaten, vorzugsweise aus Paramecium, besonders bevorzugt aus Paramecium tetraaurelia, bereit, welche bei der Exprimierung rekombinanter Peptide oder Proteine in die entsprechende Leader- oder Signalsequenz übersetzt wird, was zur Ausschleusung der rekombinant exprimierten Peptide oder Proteine aus den Ciliatenzellen führt.

Die vorliegende Erfindung wird im folgenden und anhand der Beispiele näher erläutert.

Vorliegende Arbeit beschreibt erstmals die Isolierung zweier Proteasen aus der Cathepsin-L Unterfamilie aus dem Ciliaten Paramecium (Protista). Die Sequenzierung der klonierten cDNA zeigt, daß die Übereinstimmung zu bereits beschriebenen Cathepsin-L Formen aus Mammalia und Protisten maximal 30 % beträgt, daß aber die charakteristischen Cathepsin-L-Motive sowohl in der Präproregion als auch im eigentlichen Enzym vorhanden sind. Die Proregion kodiert einen 86 Aminosäuren langen Abschnitt, der das typische ERFNIN-Motiv aufweist. Die Proregion wurde in E. coli exprimiert. Das isolierte Propeptid inhibierte effizient (im nanomolaren Bereich) das Cathepsin-L aus Paramecium. Andere Cystein-Proteasen hingegen, etwa Papain und Mammalia Cathepsin-B, - G und -H wurden selbst bei Propeptidkonzentrationen von 13 µM nicht gehemmt. Das Propeptid stellt somit einen effektiven und spezifischen Cathepsin-L Inhibitor dar. Basierend auf diesen Daten ließe sich ein potenter und hochspezifischer Inhibitor für den chemotherapeutischen Einsatz bei der Behandlung der genannten Krankheitsbilder entwickeln.

### Beispiel

### Cathepsin-L Assay

Als Substrat wurde ³²P-Phosphorylase a (ca. 5 x 10⁴ cpm/min) verwendet. Ein Testansatz (30 µl) enthielt 10 µM Substrat, 12 mM Tris/HCI (pH 7,0), 50 µM EDTA, 10 mM 2-Mercaptoethanol, 5 mM Coffeine und 6,7 µg BSA. Das Abstoppen erfolgte nach 10 minütiger Inkubation bei 30°C durch Zugabe von 200 µl Trichloressigsäure (20 % w/v). Die Radioaktivität der nicht-fällbaren Peptide wurde im Überstand nach Zentrifugation bestimmt. Eine Unit Enzymaktivität entspricht der Menge, die 1 µmol lösliches ³²P-Phosphopeptid/min freisetzt.

### Reinigung des Cathepsin-L

Als Quelle dienten Massenkulturen des Ciliaten Paramecium tetraurelia. Cathepsin-L kann sowohl aus den Zellen als auch in großen Mengen aus dem Kulturmedium gewonnen werden, da die Zellen das Enzym auch sezernieren.

Alle Reinigungsschritte wurden bei 4°C durchgeführt. Die Zellen wurden in 50 mM Tris/HCl (pH 7,0), 5 mM EDTA mittels einer French-Press homogenisiert. Zelldebris wurde über Zentrifugation (23.000 x g, 60 min; 100.000 x g, 60 min) entfernt. Der Überstand wurde auf eine mit 20 mM Tris/HCl (pH 7,0) äquilibrierte DEAE-Sepharose®-Säule aufgetragen. Etwa die Hälfte der Proteaseaktivität eluierte mit dem Durchbruch. Die Säule wurde mit 250 mM NaCl gewaschen. Die restliche Proteaseaktivität wurde mit 450 mM KCI eluiert. Danach wurden die aktiven Fraktionen über eine Sephacryl® S-100 HR-Säule gereinigt. Die Protease eluierte bei etwa 27 kDa. Die gepoolten aktiven Fraktionen wurden im Anschluß auf eine Mono-Q-Säule aufgetragen. Die Elution fand mittels eines linearen Gradienten (60 ml 100 bis 350 mM NaCl) statt. Über diesen Schritt konnten zwei aktive Proteasen (30 kDa und 33 kDa) getrennt werden. Die Reinheit wurde mittels SDS-PAGE überprüft. Gegenüber ³²P-Phosphorylase a als Substrat lag das pH-Optimum beider Isozyme bei 6,5; das Temperatur-Optimum bei 56°C. Sulfhydryl-Protease spezifische Inhibitoren (z.B. Cystatin, Leupeptin und TLCK) reduzierten die Aktivität drastisch. Dagegen zeigten Inhibitoren spezifisch für Serin-Proteasen (Aprotinin), Metallo-Proteasen (EDTA) und Asp-Proteasen (Pepstatin) keine inhibierende Wirkung. Anhand des Verdauungsmusters von Phosphorylase und BSA konnte gezeigt werden, daß es sich um zwei Endoproteinase-Isozyme handelt.

### Aminosäuresequenzierung

Die Proteine wurden aus dem SDS-Gel auf eine Polyvinyliden-difluorid Membran geblottet und die entsprechenden 30 kDa- und 33 kDa-Banden ausgeschnitten.
Für die Sequenzierung von Proteinbruchstücken wurden die Proteine vor der SDS-PAGE mit BrCN (350 µg/10 µg Protein) gespalten. Die Sequenzierung wurde auf einem Applied Biosystems Sequencer durchgeführt. Der NH₂-Terminus der 30 kDa-Bande ist: GAEVDWTDNKKVKYPAVKNQ der der 33 kDa-Bande: GAEVDXTXNK (X ist nicht geklärt). Auch die Sequenzierung der BrCN-Bruchstücke zeigte, daß es sich um identische Enzymproteine handelt, die eventuell nur unterschiedlich prozessiert werden. Hier wurde für beide Proteine folgende Sequenz ermittelt: DSAFEYVADNGLAEAKDYPYYASD. Der Vergleich mit der EMBL/Gene bank über das FASTA-Programm erbrachte hinsichtlich des NH₂-Terminus keine Übereinstimmung mit bekannten Proteinen, dagegen zeigte das Alignment des internen 24er-Peptids eindeutige Übereinstimmungen mit 19 verschiedenen Cystein-Proteasen.

### Amplifizierung und Subklonierung von Cathepsin-L

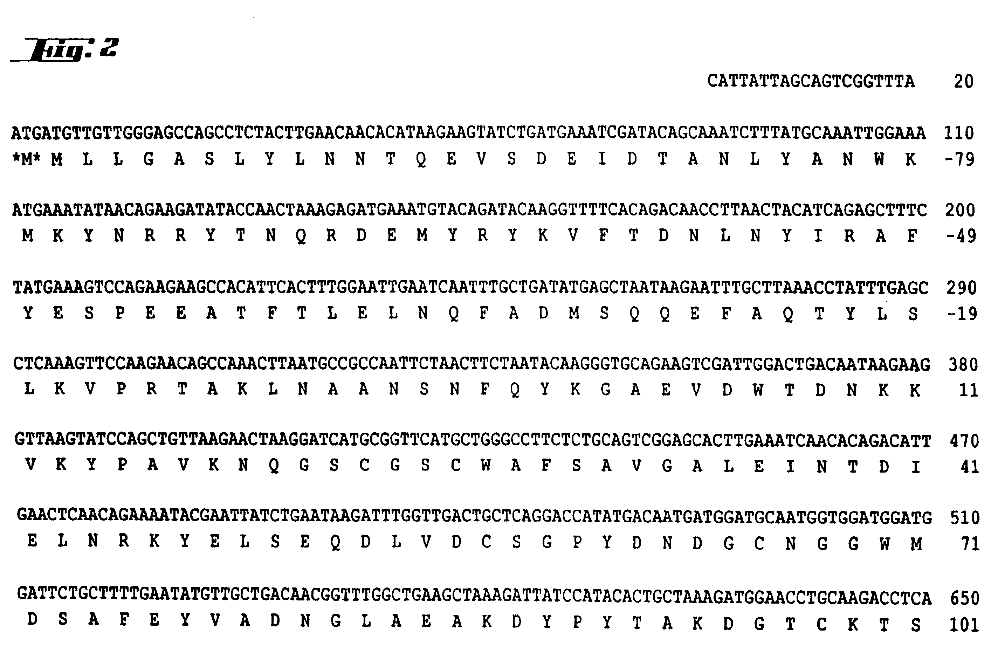

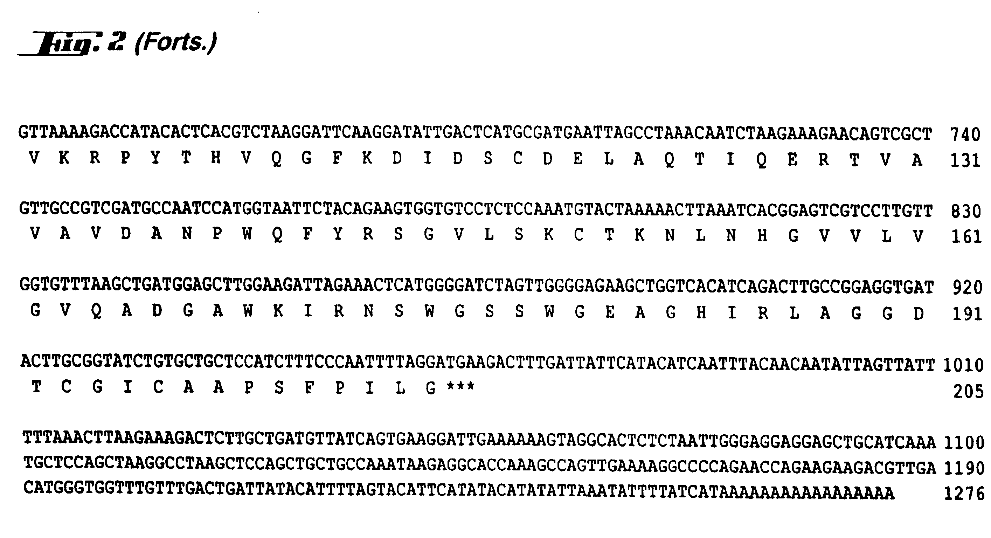

Oligonukleotide wurden hergestellt basierend auf der AS-Sequenzierung und unter Berücksichtigung des Ciliaten-codon-usage. Die verwendeten Primer waren: Primer 1 (sense) 5'-GCGGGGTACCGGWGCHGAAGTHGAYTGGACWGA-TAAYAARAARG-3' abgeleitet aus dem NH₂-terminalen Peptid GAEVDWDNKKVK und Primer 2 (antisense) 5'-TARTANGGRTARTCYTTNGC-YTC-3' abgeleitet aus der internen Peptidsequenz EAKDYPYY. Die PCR wurde in einem Perkin-Elmer Thermal-Cycler (30 Zyklen, bei 94°C, 55°C und 72°C je 1 min) durchgeführt. Mittels dieser Primer wurde aus einer Paramecium-cDNA-Bibliothek ein 275 bp langes Fragment amplifiziert. Über die Sequenzierung dieses DNA-Fragments konnte eindeutig die Ähnlichkeit zu Cathepsin-L belegt werden. So beinhaltete das PCR-Fragment die zwei stark konservierten Regionen GCNGG und CGCSWA. Aus der cDNA-Bank wurden über das 275 bp-Fragment zwei Klone mit einem Insert von 1,3 kB identifiziert. Die Sequenzierung zeigte, daß sie identische offene Laserraster enthielten, die ein Protein mit 313 Aminosäuren mit einem errechneten MG von 35.031 Da kodierten (Fig. 2). Die deduzierte Aminosäuresequenz stimmte mit der beim Edman-Abbau ermittelten überein.

Das konservierte ERFNIN-Motiv im Propeptid (EX₃RX₂(V/I)FX₂NX₃IX₃N) charakterisiert das Enzym als Cathepsin-H oder -L. Während Cathepsin-H als Exoprotease charakterisiert ist, wird Cathepsi-L als effiziente Endoprotease klassifiziert. Die Identifizierung der hier beschriebenen Proteasen als Endoproteasen, legt nahe, daß es sich in der Tat um Cathepsin-L Formen handelt. Die Übereinstimmung des Paramecium-Cathepsin-L mit verschiedenen Mammalia-Formen beträgt maximal 35 % (Tab. 1). Auch im Vergleich zur Cystein-Protease aus Tetrahymena beträgt die Übereinstimmung lediglich 30 %.

**Tabelle 1**

| Cathepsine und Proteasen im Vergleich zu Paramecium-Cathepsin L | SWISSPROT accession No. | % Identität zu | |
|---|---|---|---|
| | | reife Proteasen | Proregionen |
| Type-L Ratte | P07154 | 35 | 21 |
| Cystein Protease Tetrahymena | L03212 | 30 | 23 |
| Type-H Ratte | P00786 | 30 | 19 |
| Type-S Ratte | Q02765 | 31 | 19 |
| Type-B human | P07858 | 21 | 12 |

### cDNA-Bibliothek-Screening

Unter Verwendung von ³²P-markierten PCR-Fragmenten wurde in der cDNA-Bibliothek nach entsprechenden Klonen gescreent. Die so identifizierten zwei Klone wurden über Southern-blot-Verfahren analysiert. Beide codierten eine identische Preprocathepsin-L-Protease.

### Bakterielle Expression des Cathepsin-L-Propeptids

Das klonierte Gen enthält eine potentielle Propeptidregion von AS -1 bis -86. Der offene Leserahmen enthält fünf universelle TAA Stopcodons, die bei Paramecium für Q codieren. Vor der Expression wurden sie über "site-directed" Mutagenese in CAA (codiert Q) geändert.

Das die Propeptidregion enthaltende DNA-Fragment wurde über PCR amplifiziert und für die Expression in den hitzeinduzierbaren Vektor pEV41C eingebracht, der zusätzlich ein hexa-His tag enthielt. Die für die PCR verwendeten Primer waren 5'-AGGTCGTCATATGAATCTTTATGCAAATTGG (sense) und 5'-ATCCT-CGAGTCACTTGTATTGGAAGTTAG (antisense). Nach der Transformation wurde das Propeptid in E. coli Stamm 2136 exprimiert. Die Expression wurde induziert durch Zugabe von LBₛₘₚ-Medium, vorgewärmt auf 42°C.

Nach der Ernte wurden die Zellen homogenisiert und die Zelldebris durch Zentrifugation abgetrennt. Der Überstand wurde über eine Ni-Affinitäts-Säule (Qiagen) gereinigt. Die Elution des Proteins wurde mittels 20 mM Tris/HCl (pH 7,5), 8,6 % Glycerin, 200 mM NaCl und 500 mM Imidazol durchgeführt. Dabei eluierte erwartungsgemäß ein Protein mit einer Größe von 13,6 kDa.

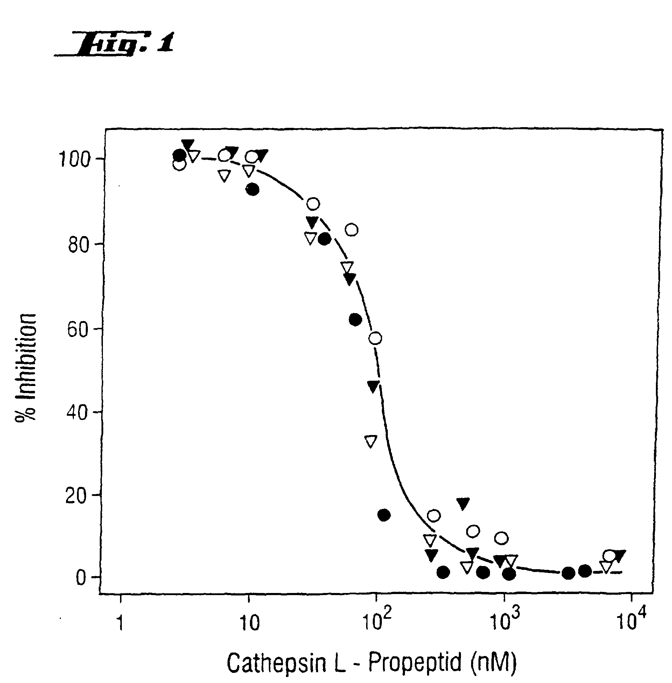

Im Hemmtest hemmte das Propeptid bereits in einer Konzentration von 60 nM das 30 kDa Cathepsin-L Isozym aus Paramecium zu 50 % (Fig. 1). Andere Proteasen (Papain, Cathepsin-H aus humaner Leber, Cathepsin-B aus Rinderniere, Cathpsin-G aus Leukocyten) wurden selbst bei Propeptidkonzentrationen von 13 µM nicht inhibiert.

## Patentansprüche

1. Präproform des Cathepsin-L, erhältlich aus einem Ciliaten, entsprechend der Aminosäuresequenz gemäß Figur 2.

2. Präproprotein nach Anspruch 1, **dadurch gekennzeichnet, dass** der Ciliat *Paramecium tetraurelia* ist.

3. DNA-Sequenz, kodierend für ein Präproprotein gemäß einem der Ansprüche 1 oder zwei.

4. DNA-Sequenz, entsprechend Figur 2.

5. Antisense-Strang der DNA-Sequenz gemäß Anspruch 4.

6. Aminosäuresequenz entsprechend Figur 2.

7. Präsequenz, entsprechend den Basen Nr. 1 bis 86 der DNA-Sequenz gemäß Anspruch 4.

8. Antisense-Strang der DNA-Sequenz gemäß Anspruch 7.

9. Kodierende Präsequenz, entsprechend den Basen Nr. 21 bis 86 der DNA-Sequenz gemäß Anspruch 4.

10. DNA-Sequenz nach Anspruch 9, **dadurch gekennzeichnet, dass** sämtliche TAA-Codons durch solche Codons ersetzt sind, die in dem entsprechenden Expressionssystem für Q codieren.

11. DNA-Sequenz nach Anspruch 10, **dadurch gekennzeichnet, dass** sämtliche TAA-Codons durch CAA-Codons ersetzt sind.

12. Antisense-Strang der DNA-Sequenz gemäß einem der Ansprüche 9-11.

13. Aminosäuresequenz entsprechend den Aminosäuren Nr. -108 bis -87 gemäß Anspruch 6.

14. Proregion der DNA-Sequenz gemäß Anspruch 4, entsprechend den Basen Nr. 87 bis 347.

15. DNA-Sequenz nach Anspruch 14, **dadurch gekennzeichnet, dass** sämtliche TAA-Codons durch solche Codons ersetzt sind, die in dem entsprechenden Expressionssystem für Q codieren.

16. DNA-Sequenz nach Anspruch 15, **dadurch gekennzeichnet, dass** sämtliche TAA-Codons durch CAA-Codons ersetzt sind.

17. Antisense-Strang der DNA-Sequenz gemäß einem der Ansprüche 14-16.

18. Propeptid der Aminosäuresequenz gemäß Anspruch 6, entsprechend den Aminosäuren Nr. -86 bis -1.

19. Cathepsin-L, erhältlich aus Ciliaten, entsprechend den Aminosäuren Nr. 1 bis 205 gemäß Anspruch 6.

20. Cathepsin-L gemäß Anspruch 19, **dadurch gekennzeichnet, dass** der Ciliat *Paramecium tetraurelia* ist.

21. DNA-Sequenz, entsprechend den Basen Nr. 348 bis 1276 der DNA-Sequenz gemäß Anspruch 4.

22. Kodierende DNA-Sequenz, entsprechend den Basen Nr. 348 bis 965 der DNA-Sequenz gemäß Anspruch 4.

23. DNA-Sequenz nach einem der Ansprüche 21 oder 22, **dadurch gekennzeichnet, dass** sämtliche TAA-Codons durch solche Codons ersetzt sind, die in dem entsprechenden Expressionssystem für Q codieren.

24. DNA-Sequenz nach Anspruch 23, **dadurch gekennzeichnet, dass** sämtliche TAA-Codons durch CAA-Codons ersetzt sind.

25. Antisense-Strang der DNA-Sequenz gemäß einem der Ansprüche 21-24.

26. Verfahren zur Herstellung des Cathepsin-L gemäß einem der Ansprüche 19 oder 20, **dadurch gekennzeichnet, dass** man Ciliaten in einem geeigneten Medium kultiviert und man anschließend das Cathepsin-L isoliert.

27. Verfahren nach Anspruch 26, **dadurch gekennzeichnet, dass** man das Cathepsin-L aus den Ciliaten-Zellen isoliert.

28. Verfahren nach Anspruch 26, **dadurch gekennzeichnet, dass** man das Cathepsin-L aus dem Medium isoliert.

29. Verfahren nach einem der Ansprüche 26 bis 28, **dadurch gekennzeichnet, dass** der Ciliat *Paramecium* ist.

30. Verfahren nach Anspruch 29, **dadurch gekennzeichnet, dass** der Ciliat *Paramecium tetraurelia* ist.

31. Cathepsin-L gemäß einem der Ansprüche 19 oder 20 zur Anwendung als Arzneimittel.

32. Verwendung des Cathepsin-L gemäß einem der Ansprüche 19 oder 20 zur Herstellung eines Arzneimittels zur Behandlung von Wunden.

33. Wundsalbe, enthaltend Cathepsin-L gemäß einem der Ansprüche 19 oder 20.

34. Verwendung des Cathepsin-L gemäß einem der Ansprüche 19 oder 20 zur Identifizierung eines Cathepsin-L-Inhibitors.

35. Cathepsin-L-Propeptid gemäß Anspruch 18 zur Anwendung als Arzneimittel.

36. Verwendung des Cathepsin-L-Propeptids gemäß Anspruch 18 zur Herstellung eines Arzneimittels zur Behandlung von inflammatorischen Erkrankungen.

37. Verwendung des Cathepsin-L-Propeptids gemäß Anspruch 18 zur Herstellung eines Arzneimittels zur Behandlung von metastasierenden Tumoren.

38. Verwendung des Cathepsin-L-Propeptids gemäß Anspruch 18 zur Herstellung eines Arzneimittels zur Behandlung von Infenktionen mit parasitären Protozoen.

39. Verwendung des Cathepsin-L-Propeptids gemäß Anspruch 18 zur Herstellung eines Arzneimittels zur Behandlung von Osteoporose.

40. Verfahren zur Herstellung des Cathepsin-L gemäß einem der Ansprüche 19 oder 20, **dadurch gekennzeichnet, dass** man unter Verwendung einer DNA-Sequenz gemäß Anspruch 4 das Cathepsin-L in einem heterologen Expressionssystem exprimieren läßt.

41. Verfahren zur Herstellung des Cathepsin-L-Propeptids gemäß Anspruch 18, **dadurch gekennzeichnet, dass** man unter Verwendung einer DNA-Sequenz gemäß Anspruch 14 das Cathepsin-L-Propeptid in einem heterologen Expressionssystem exprimieren läßt.

42. Verfahren nach Anspruch 40 oder 41, **dadurch gekennzeichnet, dass** das Expressionssystem *Escherichia coli* ist.

43. Verfahren nach Anspruch 42, **dadurch gekennzeichnet, dass** eine DNA-Sequenz gemäß Anspruch 4 oder 14 mittels eines hitzeinduzierbaren Vektors in *E. coli* eingeschleust wird.

44. Verwendung der Präsequenz gemäß einem der Ansprüche 7 oder 9 zur Translation in eine Leadersequenz gemäß Anspruch 13, welche als Signal zur Ausschleusung rekombinant exprimierter Peptide oder Proteine in Ciliaten dient.

45. Verwendung des Cathepsin-L-Propeptids gemäß Anspruch 18 oder von Teilen desselben zur Herstellung eines Medikamentes zur Inhibition von Cathepsin-L.

## Claims

1. A prepro form of cathepsin L, obtainable from a ciliate, according to the amino acid sequence as depicted in Fig. 2.

2. A prepro protein as claimed in claim 1, wherein the ciliate is Paramecium tetraurelia.

3. A DNA sequence encoding a prepro protein as claimed in one of claims 1 to 2.

4. The DNA sequence shown in Fig. 2.

5. The antisense strand of the DNA sequence according to claim 4.

6. The amino acid sequence shown in Fig. 2.

7. The presequence corresponding to bases Nos. 1 to 86 of the DNA sequence according to claim 4.

8. The antisense strand of the DNA sequence as claimed in claim 7.

9. Coding presequence corresponding to bases Nos. 21 to 86 of the DNA sequence according to claim 4.

10. A DNA sequence as claimed in claim 9, wherein all the TAA codons are replaced with those codons which encode Q in the corresponding expression system.

11. A DNA sequence as claimed in claim 10, wherein all the TAA codons are replaced with CAA codons.

12. An antisense strand of the sequence as claimed in one of claims 9 to 11.

13. The amino acid sequence corresponding to amino acids Nos. -108 to -87 according to claim 6.

14. The pro region of the DNA sequence as claimed in claim 4, according to bases Nos. 87 to 347.

15. DNA sequence as claimed in claim 14, wherin all the TAA-codons are replaced with those codons which a code Q in the corresponding expression system.

16. DNA sequence as claimed in claim 15, wherein all the TAA codons are replaced with CAA codons.

17. An antisense strand of the DNA sequence as claimed in one of claims 14-16.

18. Propeptide of the amino acid sequence according to claim 6, corresponding to amino acids Nos. -86 to -1.

19. Cathepsin L, obtainable from ciliates according to amino acids Nos. 1 to 205 according to claim 6.

20. Cathepsin L as claimed in claim 19, wherein the ciliate is Paramecium tetraurelia.

21. The DNA sequence corresponding to bases Nos. 348 to 1276 according to claim 4.

22. The coding DNA sequence, corresponding to bases Nos. 348 to 965 of the DNA-sequence according to claim 4.

23. A DNA sequence as claimed in one of claims 21 or 22, wherein all the TAA codons are replaced with those codons which encode Q in the corresponding expression system.

24. A DNA sequence as claimed in claim 23, wherein all the TAA codons are replaced with CAA codons.

25. An antisense strand of the DNA-sequence as claimed in one of claims 21 to 24.

26. A process for preparing the cathepsin L as claimed in one of claims 19 or 20 **characterized in** culturing ciliates in a suitable medium and subsequently isolating the cathepsin L.

27. The process as claimed in claim 26, wherein the cathepsin L is isolated from the ciliate cells.

28. The process as claimed in claim 26, wherein the cathepsin L is isolated from the medium.

29. The process as claimed in one of claims 26 to 28, wherein the ciliate is Paramecium.

30. The process as claimed in claim 29, wherein the ciliate is Paramecium tetraurelia.

31. A cathepsin L as claimed in one of claims 19 or 20 for use as a pharmaceutical.

32. The use of cathepsin L as claimed in one of claims 19 or 20 for preparing a pharmaceutical for treating wounds.

33. A wound ointment which comprises cathepsin L as claimed in one of claims 19 or 20

34. The use of cathepsin L as claimed in one of claims 19 or 20 for identifying a cathepsin L inhibitor.

35. A cathepsin L propeptide as claimed in claim 18 for use as a pharmaceutical.

36. The use of the cathepsin L propeptide as claimed in claim 18 for preparing a pharmaceutical for treating inflammatory diseases.

37. The use of the cathepsin L propeptide as claimed in claim 18 for preparing a pharmaceutical for treating metastasizing tumors.

38. The use of the cathepsin L propeptide as claimed in claim 18 for preparing a pharmaceutical for treating infections with parasitic protozoa.

39. The use of the cathepsin L propeptide as claimed in claim 18 for preparing a pharmaceutical for treating osteoporosis.

40. A process for preparing cathepsin L as claimed in one of claims 19 or 20, **characterized in** expressing the cathepsin L in a heterologous expression system using a DNA sequence as claimed in claim 4.

41. A process for preparing the cathepsin L propeptide as claimed in claim 18, **characterized in** expressing the cathepsin -L- propeptide in a heterologous expression system using a DNA sequence as claimed in claim 14.

42. The process as claimed in claim 40 or 41, wherein the expression system is E. coli.

43. The process as claimed in claim 42, wherein a DNA sequence as claimed in claim 4 or 14 is introduced into E. coli using a heat-inducible vector.

44. The use of the presequence as claimed in one of claims 7 or 9 for translating into a leader sequence as claimed in claim 13, which leader sequence serves as the signal for secreting recombinantly expressed peptides or proteins in ciliates.

45. The use of the cathepsin L propeptide as claimed in claim 18, or of parts thereof, for preparing a medicament for inhibition of Cathepsin L.

## Revendications

1. Préproforme de la cathepsine-L, pouvant être obtenue à partir d'un cilié, correspondant à la séquence d'aminoacides selon la figure 2.

2. Préproprotéine selon la revendication 1 **caractérisée en ce que** le cilié est *Paramecium tetraurelia*.

3. Séquence d'ADN codant une préproprotéine selon l'une des revendications 1 ou 2.

4. Séquence d'ADN correspondant à la figure 2.

5. Brin antisens de la séquence d'ADN selon la revendication 4.

6. Séquence d'aminoacides correspondant à la figure 2.

7. Préséquence correspondant aux bases n°. 1 à 86 de la séquence d'ADN selon la revendication 4.

8. Brin antisens de la séquence d'ADN selon la revendication 7.

9. Préséquence codante, correspondant aux bases n°. 21 à 86 de la séquence d'ADN selon la revendication 4.

10. Séquence d'ADN selon la revendication 9 **caractérisée en ce que** tous les codons TAA sont remplacés par des codons qui codent Q dans le système d'expression correspondant.

11. Séquence d'ADN selon la revendication 10 **caractérisée en ce que** tous les codons TAA sont remplacés par des codons CAA.

12. Brin antisens de la séquence d'ADN selon l'une des revendications 9-11.

13. Séquence d'aminoacides correspondant aux aminoacides n°. -108 à -87 selon la revendication 6.

14. Prorégion de la séquence d'ADN selon la revendication 4, correspondant aux bases n°. 87 à 347.

15. Séquence d'ADN selon la revendication 14 **caractérisée en ce que** tous les codons TAA sont remplacés par des codons qui codent Q dans le système d'expression correspondant.

16. Séquence d'ADN selon la revendication 15 **caractérisée en ce que** tous les codons TAA sont remplacés par des codons CAA.

17. Brin antisens de la séquence d'ADN selon l'une des revendications 14-16.

18. Propeptide de la séquence d'aminoacides selon la revendication 6, correspondant aux aminoacides n°. -86 à -1.

19. Cathepsine-L pouvant être obtenue à partir de ciliés, correspondant aux aminoacides n°. 1 à 205 selon la revendication 6.

20. Cathepsine-L selon la revendication 19 **caractérisée en ce que** le cilié est *Paramecium tetraurelia*.

21. Séquence d'ADN correspondant aux bases n°. 348 à 1276 de la séquence d'ADN selon la revendication 4.

22. Séquence d'ADN codante correspondant aux bases n°. 348 à 965 de la séquence d'ADN selon la revendication 4.

23. Séquence d'ADN selon l'une des revendications 21 ou 22 **caractérisée en ce que** tous les codons TAA sont remplacés par des codons qui codent Q dans le système d'expression correspondant.

24. Séquence d'ADN selon la revendication 23 **caractérisée en ce que** tous les codons TAA sont remplacés par des codons CAA.

25. Brin antisens de la séquence d'ADN selon l'une des revendications 21-24.

26. Procédé de préparation de la cathepsine-L selon l'une des revendications 19 ou 20 **caractérisé en ce que** l'on cultive des ciliés dans un milieu approprié puis on isole la cathepsine-L.

27. Procédé selon la revendication 26 **caractérisé en ce que** l'on isole la cathepsine-L à partir des cellules de ciliés.

28. Procédé selon la revendication 26 **caractérisé en ce que** l'on isole la cathepsine-L à partir du milieu.

29. Procédé selon l'une des revendications 26 à 28 **caractérisé en ce que** le cilié est *Paramecium.*

30. Procédé selon la revendication 29 **caractérisé en ce que** le cilié est *Paramecium tetraurelia*.

31. Cathepsine-L selon l'une des revendications 19 ou 20 destinée à être utilisée comme médicament.

32. Utilisation de la cathepsine-L selon l'une des revendications 19 ou 20 pour la production d'un médicament pour le traitement des plaies.

33. Onguent vulnéraire contenant de la cathepsine-L selon l'une des revendications 19 ou 20.

34. Utilisation de la cathepsine-L selon l'une des revendications 19 ou 20 pour l'identification d'un inhibiteur de cathepsine-L.

35. Propeptide de cathepsine-L selon la revendication 18 destiné à être utilisé comme médicament.

36. Utilisation du propeptide de cathepsine-L selon la revendication 18 pour la production d'un médicament pour le traitement des maladies inflammatoires.

37. Utilisation du propeptide de cathepsine-L selon la revendication 18 pour la production d'un médicament pour le traitement des tumeurs métastasiantes.

38. Utilisation du propeptide de cathepsine-L selon la revendication 18 pour la production d'un médicament pour le traitement des infections par des protozoaires parasitaires.

39. Utilisation du propeptide de cathepsine-L selon la revendication 18 pour la production d'un médicament pour le traitement de l'ostéoporose.

40. Procédé de production de la cathepsine-L selon l'une des revendications 19 ou 20 **caractérisé en ce qu'**en utilisant une séquence d'ADN selon la revendication 4 on fait s'exprimer la cathepsine-L dans un système d'expression hétérologue.

41. Procédé de production du propeptide de cathepsine-L selon la revendication 18 **caractérisé en ce qu'**en utilisant une séquence d'ADN selon la revendication 14 on fait s'exprimer le propeptide de cathepsine-L dans un système d'expression hétérologue.

42. Procédé selon la revendication 40 ou 41 **caractérisé en ce que** le système d'expression est *Escherichia coli.*

43. Procédé selon la revendication 42 **caractérisé en ce qu'**une séquence d'ADN selon la revendication 4 ou 14 est introduite dans *E. coli* au moyen d'un vecteur inductible par la chaleur.

44. Utilisation de la préséquence selon l'une des revendications 7 ou 9 pour la traduction en une séquence leader selon la revendication 13 qui sert de signal pour la sécrétion de peptides ou protéines exprimés par recombinaison dans des ciliés.

45. Utilisation du propeptide de cathepsine-L selon la revendication 18 ou de parties de celui-ci pour la production d'un médicament pour l'inhibition de la cathepsine-L.
